# EUROPEAN PATENT APPLICATION

(11) **EP 2 088 155 A1**
(43) Date of publication of application: **12.08.2009**
(21) Application number: 09004681.4
(22) Date of filing: 28.12.2005
(51) Int. Cl.: C07K 14/16, A61K 39/21

(54) **Hetero-oligomeric HIV envelope proteins**

(30) Priority: 29.12.2004 US 640329 P
(62) Divisional of application: 05855755.4
(71) Applicant: SEATTLE BIOMEDICAL RESEARCH INSTITUTE, Seattle WA 98109-5219 (US)
(72) Inventor: Stamatatos, Leonidas, Seattle, WA 98103 (US)
(74) Representative: Bizley, Richard Edward

(57) **Abstract**

In one aspect, the invention provides an HIV envelope heterotrimer comprising at least two different Env glycoprotein monomers. In some heterotrimers, at least two Env glycoprotein monomers are from different HIV isolates, for example, different HIV-1 isolates. Heterotrimers may contain Env glycoprotein monomers from HIV isolates belonging to the same clade or to different clades or both. At least one of the Env glycoprotein monomers in a heterotrimer of the invention may be modified in a way that alters its amino acid composition. In another aspect, the invention provides methods for inducing an immune response in a vertebrate host against HIV or an HIV-infected cell, comprising administering to a vertebrate host a prophylactically or therapeutically effective amount of a composition comprising an HIV envelope heterotrimer.

SEQUENCE LISTING

<110> Seattle Biomedical Research Institute

<120> Hetero-oligomeric HIV Envelope Proteins

<130> P100066EP02

<150> US 60/640,329
<151> 2004-12-29

<160> 3

<170> PatentIn version 3.2

<210> 1
<211> 18
<212> DNA
<213> Artificial Sequence

<220>
<223> Synthetic primer

<400> 1
catcaccatc accatcac 18

<210> 2
<211> 9
<212> PRT
<213> Artificial Sequence

<220>
<223> Synthetic Protein for FLAG tag

<400> 2

Asp Tyr Lys Asp Asp Asp Asp Lys Lys
1               5

<210> 3
<211> 27
<212> DNA
<213> Artificial Sequence

<220>
<223> Synthetic Primer for FLAG tag

<400> 3
gattacaagg atgacgatga caaaaag 27

## Description

### CROSS-REFERENCE TO RELATED APPLICATION

This application claims the benefit of U.S. Provisional Application No. 60/640,329, filed December 29, 2004, which is incorporated herein by reference.

### STATEMENT OF GOVERNMENT LICENSE RIGHTS

The U.S. Goverment has a paid-up license in this invention and the right in limited circumstances to require the patent owner to license others on reasonable terms as provided for by the terms of Grant No. R01 AI047708 awarded by the National Institutes of Health.

### FIELD OF THE INVENTION

This invention relates to recombinant hetero-oligomeric Human Immunodeficiency Virus (HIV) envelope proteins and their uses, for example, as vaccines.

### BACKGROUND OF THE INVENTION

It is increasingly evident that an effective vaccination methodology against HIV should elicit both neutralizing antibodies (NAbs) and cell-mediated anti-viral responses. Although the protective role of *de novo* neutralizing antibody responses early following of HIV/SIV infection remains controversial, there is abundant evidence that if NAbs are present at the time of viral exposure, they would offer a significant protective benefit to the vaccinee.

Previous studies indicate that in order for a vaccine against HIV to be effective it should elicit not only high titers of NAbs but also antibodies that can neutralize as a diverse as possible panel of primary HIV isolates. The generation of high titers of NAbs during immunization depends to a large extent on the immunization methodology. The development, however, of cross-reactive NAbs depends primarily on the structure of the immunogen (a derivative of the HIV envelope glycoprotein). Not only must the immunogen contain neutralization epitopes that are conserved among heterologous primary HIV isolates, but also, these epitopes must be exposed on the surface of the immunogen in a way that induces an efficient stimulation of the immune system.

Despite the significant amino acid variability of the HIV envelope, certain domains of this protein must remain structurally unaltered over time and across subtypes in order for this viral protein to retain its functionality. Among these domains are those participating in the interaction of the HIV envelope with the receptor and coreceptor molecules on the target cell membranes. Additional conserved regions are present on the extracellular portion of gp41 (for HIV-1; gp36 for HIV-2), which is critical for the fusion step between the viral envelope and the target cell membrane, and within the V3 loop, which contains critical determinants for envelope function. These structurally and functionally conserved envelope regions are accessible to antibodies. For instance, monoclonal antibodies (MAbs) that can neutralize many, but not all, diverse primary isolates have been isolated (rarely) from HIV-infected patients and recognize epitopes located in these conserved regions (Burton et al., 1994; Calarese et al., 2003; Conley et al., 1994; Moulard et al., 2002; Muster et al., 1993; Pantophlet et al., 2003; Purtscher et al., 1996; Purtscher et al., 1994; Sanders et al., 2002a; Scanlan et al., 2002; Thali et al., 1993; Trkola et al., 1996; Xiang et al., 2002; Zwick et al., 2001).

One of the major problems racing the "rational design" approach to engineer HIV envelope immunogens based on information derived from the structural and immunochemical analysis of the HIV envelope is the current inability to predict the immunogenic properties of HIV envelope proteins by examining their antigenic structure. For example, gp120 monomeric proteins do not elicit NAbs against heterologous primary HIV-1 isolates, even though conserved neutralization epitopes are present on such constructs and antibodies bind to them (Boddows et al., 1999; Bures et al., 2000; Cho et al., 2001; Haigwood et al., 1992; Hanson, 1994; Mascola et al., 1996; Nara et al., 1988; VenCott et al., 1999). The antibodies elicited by monomeric gp120 primarily recognize linear epitopes within the variable regions of gp120, such as the third variable region (V3 loop), as well as in the first and second variable regions, the V1 and V2 loops, respectively (Stephens et al., 1992; VanCott et al., 1999) and have a limited breadth of neutralizing activity against primary HIV-1 isolates (Bures et al., 2000; Hanson; 1994; Mascola et al., 1996) even though they do react with envelope glycoproteins from such isolates (Gorse et al., 1999).

A currently prevalent belief in the field of HIV envelope immunogen design is that in order for HIV envelope immunogens to elicit more relevant NAbs, they need to be in a form similar to that of virion-associated envelopes, the target of Nabs, that is, a trimeric form. In fact, intact virion-associated envelopes have been used as immunogens (Grovit-Ferbas et al., 2000; Lifson et al., 2004). Methods have been developed to generate stable soluble trimeric forms of the HIV envelope, comprising the gp120 subunit (gp125 for HIV-2) and the extracellular region of the gp41 subunit (gp36 for HIV-2), as immunogens (termed gp140 for HIV-1) (Binley et al., 2000; Binley et al., 2002; Buckner et al., 2004; Farzan et al., 1998; Sanders et al., 2002b; Schulke et al., 2002; Srivastava et al., 2003a; Yang et al., 2000). Gp140 immunogens appear to be more effective than monomeric gp120 proteins in eliciting NAbs, including cross-reactive NAbs (Dong et al., 2003; Earl et al., 2001; Yang et al., 2001; Yang et al., 2004a). However, despite the potential similarities between soluble trimeric gp140 and vition-associated gp160 envelope proteins, the engineering of such soluble gp140 proteins is not yet optimal, and the exposure of neutralization epitopes is less than optimal on either form.

Results from the crystallographic analysis of the HIV envelope indicate that the variable regions of the HIV envelope glycoprotein are the most accessible regions on the trimeric envelope (Kwong et al., 1998; Wyatt et al., 1998; Wyatt et al., 1995).. These results are supported by numerous immunochemical studies of virion-associated envelope molecules and soluble trimeric gp140 proteins. Also, the HIV envelope protein is heavily glycosylated in and around the variable regions. The position of the variable regions and their glycosylation pattern limits the accessibility of conserved regions to antibodies, even though certain, rare, broadly reactive NAbs efficiently access their conserved epitopes on the oligomeric HIV envelope (Kwong et al., 2002). The particular organization of the variable regions and glycosylation sites on oligomeric HIV envelope constructs, limits the immunogenicity of the conserved regions. It has been proposed that specific modifications need to be introduced within the variable regions of the HIV envelope in order to dampen their immunogenicity and in parallel enhance (directly or indirectly) the immunogenicity of the conserved regions (Coffin, 1986). Such modifications include the removal of N-linked glycosylation sites (NLGS) and the deletion of segments from the variable regions.

Early attempts to alter the immunogenic properties of the HIV envelope-based immunogens were met with little success, most likely due to the use of gp120-derived proteins as immunogens. As more information was generated over time on the antigenic and oligomeric structure of the HIV envelope, the design of HIV envelope immunogens became more sophisticated. Deletions introduced in the V1, V2, and V3 regions of cell-associated HIV envelopes (derived from the X4-tropic and lab-adapted HxB2 isolate) were shown to increase the binding of anti-CD4-binding site antibodies, such as F105 (Wyatt et al., 1995; Wyatt et al., 1993). Unfortunately, HxB2-derived gp120 or gp140 immunogens lacking the V1, V2, and V3 regions, or DH12-derived gp160/gp120 immunogens lacking the V1 and V2, or the V1, V2, and V3 regions, failed to elicit NAbs even against the respective homologous viruses (Kim et al., 2043; Lu et al., 1998). Most likely, extensive deletions alter the proper immunogenic structure of the HIV envelope proteins. A less disruptive modification of the V2 region on the R5-tropic SF162 envelope, has been shown by us to elicit strong NAbs against the parental SF162 virus and against certain heterologous HIV-1 isolates (Barnett et al., 2001). More recently, others reported that HIV-1 envelope immunogens with specifically modified V3 loops, elicit cross-reactive NAbs (Gzyl et al., 2004; Lorin et al., 2004; Yang et al., 2004b).

Another type of modification consists of altering the glycosylation pattern of the HIV envelopes. In the Simian Immunodeficiency Virus (SIV) model, it was demonstrated that viruses Envs that were specifically modified to lack certain NLGS from the V1 loop were capable of infecting macaques and in eliciting high titers of NAbs against the homologous, parental SIV virus that expresses the unmodified Env (Reitter et al., 1998). The animals infected with these modified viruses elicited higher titers of NAbs against the parental virus than those elicited in animals infected with the parental virus itself. These results suggested that elimination of NLOS from the V1V2 region of

SIV alters the exposure and increases the immunogenicity of neutralization epitopes.

However, these epitopes must not be conserved among SIV strains, since the antibodies elicited by the above-mentioned deglycosylated SIV envelopes had a very narrow breadth of neutralizing activity. It has been reported that the elimination of NLGS from the V1 and V2 loops from 89.6 HIV-1 envelope immunogens results in the generation of homologous, but not heterologous, NAbs (Quinones-Kochs et al., 2002). Similarly, immunogens lacking specific NLGS from the V3 loop of HIV BRU, elicit NAbs against the parental but not heterologous HIV-1 isolates (Bolmstedt et al., 2001; Schonning et al., 1996). Recently, it was reported that removal of NLGS from the immunologically "silent" face of the HIV envelope exposes numerous conserved neutralization epitopes located both in gp120 and in gp41 (McCaffrey et al., 2004). Thus, HIV envelope immunogens containing modifications in the V4, C4, and V5 regions are likely to be more effective in eliciting cross-reactive NAbs than constructs containing mutations only in the V1, V2 and V3 regions.

Overall, incremental but significant advances in the design of gp140 immunogens have been made in recent years, but further improvement is required to ameliorate the potential of these constructs to elicit cross-reactive NAbs (Barnett et al., 2001; Dong et al., 2003; Gzyl et al., 2004; Jeffs et al., 2002; Lorin et al., 2004; Yang et al., 2001; Yang et al., 2004b). There is a need in the art for immunogens that elicit a heterologous immune response to HIV, such as broadly reactive NAbs. The present invention addresses this need and others.

### SUMMARY OF THE INVENTION

A first aspect of the invention provides an HIV envelope heterotrimer comprising at least two different Env glycoprotein monomers. The Env glycoprotein monomers in the heterotrimers of the invention may be mature Env glycoprotein monomers or soluble Env glycoprotein monomers. In some embodiments, at least two Env glycoprotein monomers are from different HIV isolates, for example, different HIV-1 isolates. In a heterotrimer comprising Env glycoprotein monomers from different HIV isolates, the different HIV isolates may belong to the same clade or to different clades or both.
At least one of the Env glycoprotein monomers in a heterotrimer of the invention may be modified in a way that alters its amino acid composition. For example, a monomer may be modified by removing one or more variable loop regions, or by changing one or more of its glycosylation sites.
In a second aspect, the invention provides compositions comprising an HIV envelope heterotrimer and a pharmaceutically acceptable carrier, wherein the HIV envelope heterotrimer comprises at least two different Env glycoprotein monomers. In some embodiments, the compositions comprise two or more different heterotrimers.
In a third aspect, the invention provides a vaccine comprising an effective amount of an HIV envelope heterotrimer and a pharmaceutically acceptable carrier, wherein the HIV envelope heterotrimer comprises at least two different Env glycoprotein monomers. In some embodiments, the vaccine comprises two or more different heterotrimers. The vaccine may further comprise one or more adjuvants.
In a fourth aspect, the present invention provides methods for inducing an immune response in a vertebrate host against HIV or an HIV-infected cell. In some embodiments, the methods comprise the step of administering to a vertebrate host a prophylactically or therapeutically effective amount of a composition comprising an HIV envelope heterotrimer, wherein the heterotrimer comprises at least two different Env glycoprotein monomers. In some embodiments, the composition administered comprises two or more different heterotrimers. The composition may further comprise one or more adjuvants.

In some embodiments, the immune response elicited using the methods of the invention is a heterologous immune response, such that the immune response is directed to at least one HIV isolate that is different from the HIV isolate(s) represented by the Env glycoprotein monomers in the heterotrimers that were administered. In some embodiments, the heterologous immune response is a humeral immune response and provides antibodies, such as neutralizing antibodies and/or protective antibodies. In some embodiments, the heterologous immune response comprises broadly reactive neutralizing antibodies, for example, neutralizing antibodies that are directed to at least ten different HIV isolates (such as 20, 50, or 100 different HIV isolates). In some embodiments, the heterologous immune response comprises neutralizing antibodies directed to HIV isolates from different clades.

Thus, some embodiments provide methods for eliciting an immune response in a vertebrate against HIV or an HIV-infected cell, comprising the step of administering to a vertebrate host a prophylactically or therapeutically effective amount of a composition comprising an HIV envelope heterotrimer, wherein the heterotrimer comprises at least two different Env glycoprotein monomers and wherein the composition elicits an immune response to at least one HIV isolate that is different from the HIV isolate(s) represented by the Env glycoprotein monomers in the heterotrimers.

Moreover, some embodiments provide methods for preventing or treating an HIV infection, comprising the step of administering to a vertebrate host a prophylactically or therapeutically effective amount of a composition comprising an HIV envelope heterotrimer, wherein the heterotrimer comprises at least two different Env glycoprotein monomers.

The invention further provides methods for producing neutralizing antibodies and neutralizing antibodies elicited by using the heterotrimers of the invention as immunogens.

### DETAILED DESCRIPTION OF THE PREFERRED EMBODIMENT

One of the major stumbling blocks in the development of an effective vaccine against HIV is the current inability to elicit by immunization broadly reactive NAbs, that is, antibodies that block infection of diverse HIV clinical isolates. NAbs are one of several types of anti-HIV immune responses that an effective vaccine against HIV should elicit. However NAbs with any significant breadth of activity have not yet been elicited by immunization with HIV envelope protein immunogens. The HIV envelope-based immunogens tested so far in animals or humans elicit antibody responses that primarily or exclusively target viruses that express an envelope that is homologous to the vaccine.
Thus, it has not been possible to force the immune system to focus on particular regions of the HIV envelope, those that contain conserved neutralization epitopes (the target of broadly reactive NAbs).

The functional HIV Env is thought to consist of three molecules (gp160 for HIV-1; gp140 for HIV-2), Each gp160 molecule (monomer) consists of an extracellular subunit (gp120) that is non-covalently associated to a transmembrane subunit (gp41). Both gp120 and gp41 subunits are glycosylated. The three gp160 monomers of each trimer have the same amino acid sequence and the same glycosylation pattern (homotrimers). Viral as well as DNA vectors expressing these gp160 proteins have been used as immunogens for the generation of anti-HIV immune responses, in particular neutralizing antibodies (NAbs).

Mutagenesis can be used to create a soluble form of Env that is secreted. For example, the introduction of stop codon sequences immediately upstream from the transmembrane region of HIV-1 gp41 leads to the secretion of a glycoprotein termed gp140, which consists of the entire gp120 subunit and the extracellular domain of gp41. Such gp140 Env proteins have also been expressed using viral or DNA vectors and have been used as immunogens to elicit anti-HIV Nabs, as described above. Gp140 constructs can also be purified from solution and used as purified proteins to elicit anti-HIV immune responses.

Homotrimeric gp160 or gp140 immunogens have been shown to elicit anti-HIV neutralizing antibody responses of limited breadth in animals, that is, they elicit NAbs that can neutralize a handful of HIV isolates, primarily those that express an Env that is closely related (in amino acid sequence and glycosylation pattern) to the immunogen itself.

In one aspect, the present invention provides HIV Env heterotrimers comprising at least two different Env glycoprotein monomers. The term "HIV Env heterotrimer" or "heterotrimer" refers to a complex containing at least two non-identical HIV-1 or HIV-2 envelope glycoprotein monomers. The Env glycoproteins in the heterotrimers of the invention may be mature Env monomers or soluble Env monomers. As used herein, the term "mature Env monomer" refers to both a HIV-1 gp160 Env glycoprotein, comprising the HIV-1 Env gp120 subunit and the HIV-1 Env transmembrane subunit gp41, and a HIV-2 gp140 glycoprotein, comprising the HIV-2 Env gp125 subunit and the HIV-2 transmembrane subunit gp36. The term "soluble Env monomer" refers to both the soluble HIV-1 Env glycoprotein (termed gp140), comprising the HIV-1 Env gp120 subunit and the extracellular region of the HIV-1 Env gp41 subunit, and the soluble HIV-2 Env glycoprotein, comprising the HIV-2 Env gp125 subunit and the extracellular region of the HIV-2 Env gp36 subunit. The heterotrimeric nature of these HIV Env proteins are likely to result in the presentation of neutralization epitopes that differ from those on homotrimers, which may lead to the elicitation of broader neutralizing antibody responses upon immunization.

There are two distinct types of HIV, HIV-1 and HIV-2, which are distinguished by their genomic organization and their evolution from other lentiviruses. Based on phylogenetic criteria (i.e., diversity due to evolution), HIV-1 can be grouped into three groups (M, N, and O). Group M is subdivided into 11 clades (A through K). HIV-2 can be divided into six distinct phylogenetic lineages (clades A through F) (Human Retroviruses and AIDS 1998: A compilation and analysis of nucleic acid and amino acid sequences (Los Alamos National Laboratory, Los Alamos, NM, 1998, http://hiv-web.lanl.gov). Some embodiments of the invention provide heterotrimers comprising HIV-1 Env glycoprotein monomers. In some embodiments, the heterotrimers of the invention comprise HIV-2 Env glycoprotein monomers.

In some embodiments, at least two Env glycoprotein monomers in the heterotrimers of the invention are from different HIV isolates. For example, one of the Env glycoprotein monomers in a heterotrimer may be from a HIV-1 isolate and another Env glycoprotein monomer may be from an HIV-2 isolate. Similarly, one of the Env glycoprotein monomers in a heterotrimer may be from one HIV-1 isolate and another Env glycoprotein monomer may be from another HIV-1 isolate. In a heterotrimer comprising Env glycoprotein monomers from different HIV isolates, the different HIV isolates may belong to the same clade or to different clades, or both (e.g., two different isolates from one clade and one isolate from another clade). Thus, an exemplary heterotrimer of the invention comprises Env glycoprotein monomer(s) from clade A and clade B isolates (clade A/B heterotrimers), or Env glycoprotein monomer(s) from clade A and clade C isolates (clade A/C heterotrimers), or Env glycoprotein monomer(s) from clade A and clade D isolates (clade A/D heterotrimers), or monomer(s) from clade B and clade C isolates (clade B/C heterotrimers), or Env glycoprotein monomer(s) from clade B and clade D (clade B/D heterotrimers), or Env glycoprotein monomer(s) from clade C and clade D (clade C/D heterotrimers): For example, a heterotrimer may include one or two Env glycoprotein monomers from a clade B isolate (e.g., SF162) and one or two Env glycoprotein monomers from a clade A isolate (e.g., Q168, Q259, Q461, and/or Q769).

Heterotrimers comprising monomers from HIV isolates from three different clades are also within the scope of the invention (e.g., clade A/B/C heterotrimers). Amino acid sequences of Env glycoprotein monomers that may be included in a heterotrimer of the invention may be found in the literature, such as at the Los Alamos National Laboratories HIV sequence database (accessible at httip://hiv-web.lanl.gov/content/hiv-db/mainpage.html), incorporated herein by reference in their entireties.

In some embodiments, at least one of the Env glycoprotein monomers in a heterotrimer of the invention is modified in a way that alters its amino acid composition, using standard molecular biological methods. A modified Env glycoprotein monomer is a molecule that has at least one amino acid sequence difference compared to the sequence of the wildtype HIV isolate from which it was derived. For example, a monomer may be modified by removing one or more variable loop regions (e.g., V1, V2, and/or V3), for example, as described in EXAMPLES 1 and 3. The amino acid composition of one or more Env glycoproteins may also be altered to change its glycosylation pattern, for example, by adding or removing one or more glycosylation sites (e.g., N-linked glycosylation sites or O-linked glycosylation sites) compared to the wildtype isolate (see, e.g., McCaffrey et al., 2004; Saunders et aL, 2005). Thus, some embodiments of the invention provide heterotrimers comprising at least one Env glycoprotein monomer comprising a deletion of a variable loop region. Some embodiments of the invention provide heterotrimers comprising at least one Env glycoprotein monomer comprising an alteration in a glycosylation site.

In some embodiments, one or more of the Env glycoprotein monomers in the heterotrimers of the invention may be modified to enhance the stability of the heterotrimers. For example, a trimeric motif (e.g., a coiled coil) or one or more cysteine residues may be introduced into one or more monomers, as previously described (see, e.g., U.S. Patent Nos. 6,716,429 and 6,911,205, U.S. Patent Publication No. 20050089526, or Farzan et al., 1998, all of which are herein incorporated by reference).

In some embodiments, the heterotrimers of the invention comprise soluble Env glycoprotein monomers. Methods for preparing soluble Env glycoprotein are standard in the art. For example, one or more Env glycoprotein monomers may be synthesized as a fusion protein comprising a tag. Such a tag is generally present at either the aminoterminus or the carboxyterminus of the monomer. Different tags may be used with different monomers. Suitable tags include, but are not limited to, maltose binding protein (MBP), His, FLAG, Myc, GST, etc. (see, e.g., Nilson et al., 1997, in Protein expression and purification 11:1-16). The tag-monomer fusion proteins may further include a cleavage site between the tag and the monomer, thus permitting the tag to be removed enzymatically following purification. Exemplary cleavage sites include, but are not limited to sites cleaved by HCV-3C protease, Enterokinase, Factor Xa, Thrombin, H64A subtilisin, IGA protease, GST-protease 3C, ABP-protease 3C-His₆, etc.

Plasmids encoding Different Env glycoprotein monomers may be introduced into suitable host cells using standard methods. Suitable host cells include, but are not limited to, mammalian cell lines such as, for example, monkey kidney CV 1 line transformed by SV40 (COS-7); human embryonic kidney line 293; baby hamster kidney cells (BHK); Chinese hamster ovary-cells-DHFR⁺ (CHO); Chinese hamster ovary-cells DHFR-(DXB11); monkey kidney cells (CV1); African green monkey kidney cells (VERO-76); human cervical carcinoma cells (HELA); canine kidney cells (MDCK); human lung cells (W138); human liver cells (Hep G2); mouse mammary tumor (MMT 060562); mouse cell line (C127); and myeloma cell lines. Other eukaryotic expression systems using non-mammalian vector/cell line combinations can be used to produce the Env glycoprotein monomers and heterotrimers of the invention. These include, but are not limited to, baculovirus vector/insect cell expression systems and yeast shuttle vector/yeast cell expression systems. The ratio of different plasmids introduced into host cells may be adjusted to provide for the highest proportion of heterotrimers, as is well-known in the art.

In some embodiments, the heterotrimers of the invention comprise mature Env glycoprotein monomers. An exemplary method for preparing gp160 heterotrimers is described, for example, in EXAMPLES 1 and 2. Viral particles comprising heterotrimers that comprise mature Env glycoprotein monomers may be purified using methods that are standard in the art. In some embodiments, the heterotrimers of the invention may be expressed in proteoliposomes, as described, for example, in U.S. Patent No. 6,761,902, herein incorporated by reference.

In some embodiments the invention provides purified heterotrimers. Individual tagged Env glycoprotein monomers, or heterotrimers containing tagged monomers, may be purified from homotrimers using anti-tag antibodies. For example, different tags may be used for different monomers, as described in EXAMPLES 3 and 4. Heterotrimers formed from differently tagged monomers will include at least two different tags, whereas homotrimers formed from these monomers will only include one type of tag.

Heterottimers containing monomers from different isolates may be prepared and purified from homotrimers using isolate-specific, clade-specific, or HIV-specifio antibodies.

Different heterotrimers species may be separated by taking advantage of differences in size and/or glycosylation patterns of individual monomers, for example, using size exclusion chromatography. Heterotrimers containing tagged monomers may be enzymatically cleaved to remove the tag(s). Heterotrimers may be further purified, for example, using a lectin column as previously described (Srivastava et al., 2003a). In some embodiments, the purified heterotrimers may be between 75% and 100% pure, such as between 80% and 99% pure or between 85% and 98% pure. Exemplary methods for preparing and purifying gp140 heterotrimers is described, for example, in EXAMPLES 3 and 4.

In a second aspect, the invention provides compositions comprising an HIV envelope heterotrimer and a pharmaceutically acceptable carrier, wherein the HIV envelope heterotrimer comprises at least two different Env glycoprotein monomers. The makeup of the heterotrimers in this aspect of the invention is as described above. Thus, at least one of the monomers in a heterotrimer of this aspect of the invention may modified in a way that alters its amino acid composition, as described above. For example, one or more monomers may be modified by deleting one or more variable loops and/or by adding or deleting one or more glycosylation sites. In some embodiments, the heterotrimers include monomers from at least two different HIV isolates, as described above. The compositions of the invention may also comprise two or more different hoterotrimers.

The term "pharmaceutically acceptable carrier" refers to one or more carriers, excipients, diluents, or other components that may be used to facilitate the administration of the composition without producing undesirable effects. Suitable pharmaceutically acceptable carriers include, but are,not limited to, sterile water or sterile physiological salt solution, particularly phosphate buffered saline (PBS), as well known in the art. The compositions may also include one or more adjuvants. Suitable adjuvants include, but are not limited to, alum, Freund's incomplete adjuvant, Saponin, Quil A, QS21, Ribi Detox, Monophasphoryl lipid A, and nonionic block copolymers such as L-121 (Pluronic; Syntex SAF). One of skill in the art can readily determine suitable adjuvants to include to achieve the desired effect. Methods of combining adjuvants with antigens are also well-known in the art.

In a third aspect, the invention provides a vaccine comprising an effective amount of an HIV envelope heterotrimer and a pharmaceutically acceptable carrier, wherein the HIV envelope heterotrimer comprises at least two different Env glycoprotein monomers.
The makeup of the heterotrimers in this aspect of the invention is as described above. In some embodiments, the vaccine comprises two or more different heterotrimers. In some embodiments, the vaccine may further comprise one or more adjuvants, as described above. The term "effective amount" refers to an amount that is sufficient for eliciting an immune response in a vertebrate host, for example, a humeral immune response (e.g., neutralizing antibodies and/or protective antibodies). In some embodiments, the immune response elicited is a heterologous immune response, such that the immune response is directed to at least one HIV isolate that is different from the HIV isolate(s) represented by the Env glycoprotein monomers in the heterotrimers of the vaccine. For example, a heterologous immune response to a heterotrimer comprising one or two clade A isolate Q168 Env monomers (wildtype or modified) and one ore two clade B isolate SF162 Env monomers (witdtype or modified) may elicit a heterologous immune response to one or more different clade A isolates, one or more different clade B isolates, and/or one or more isolates belonging to a different clade (e.g., clade C). One of skill in the art can readily determine the ability of an immunogen to elicit a heterologous HIV immune response in a vertebrate host Exemplary methods for measuring immune responses elicited by HIV Env immunizations have been previously described (see, e.g., Barnett et al., 2001; Buckner et al., 2004; Srivastava et al., 2003b; U.S. Patent Application Publication No. 20020127238, herein incorporated by reference).

In a fourth aspect, the present invention provides methods for inducing an immune response in a vertebrate host against HIV or an HIV-infected cell. In some embodiments, the methods comprise the step of administering to a vertebrate host a prophylactically or therapeutically effective amount of a composition comprising an HIV envelope heterotrimer, wherein the heterotrimer comprises at least two different Env glycoprotein monomers. The makeup of the heterotrimers in this aspect of the invention is as described above. In some embodiments, the composition administered comprises two or more different heterotrimers. In some embodiments, the composition may further comprise one or more adjuvants, as described above.

A "prophylactically effective amount" refers to an amount that is sufficient to prevent or reduce the risk or likelihood of being infected with HIV or having an HIV-induced disease. A "therapeutically effective amount" refers to an amount that is sufficient to treat, ameliorate, or slow or stop the progression of an HIV infection or HIV-induced disease. The vertebrate host may be a mammalian host, for example a human host. The vertebrate host may be infected with HIV or at risk of being infected with HIV or having an HIV-induced disease. Thus, the methods of the invention may be used therapeutically to treat or ameliorate an HIV infection or an HIV-induced disease, or prophylactically to prevent or reduce the likelihood of HIV infection or HIV-induced disease.

The compositions or vaccines comprising an HIV envelope heterotrimer may be administered by any suitable method of administration known in the art, including, but not limited to, intradermal, subcutaneous, intramuscular, intraperitoneal, oral, ocular (e.g., as an eye spray), topical, intravaginal, and intravenous. The compositions may be provided in unit dosage form and may be prepared by any of the methods well-known in the art pharmacy. Dosage is empirically selected to achieve the desired immune response in the host, for example, an acquired and enhanced degree of protective immunity, preferably complete protection, against subsequent exposure to HIV. Dosages will vary depending on the subject and the route of administration used, and may be readily determined using routine experimentation.

The composition comprising one or more heterotrimers may be administered as part of any type of or protocol for immunizations. Such immunization protocols may include additional immunogens, which may be in the form of DNA, virus protein, and/or combinations thereof. One typical method for inducing an immune response is what is known as "prime-boost." In some embodiments, an immune response may be primed using one heterotrimer or a mixture of different heterotrimers and subsequently boosted using one heterotrimer or a mixture of different heterotrimers. The heterotrimer(s) used to prime the immune response may be different from the heterotrimer(s) used to boost the immune response. In some embodiments, DNA vectors or replicons expressing viral HIV antigens (e.g., homotrimeric antigens) may be used to prime the immune response, followed by boosting with heterotrimer(s) of the invention. Exemplary methods of immunizing vertebrate hosts with HIV antigens have been previously described (see, e.g., Barnett et al., 2001; Buckner et al., 2004; U.S. Patent Application Publication No. 20020127238, herein incorporated by reference)

In some embodiments, the immune response elicited using the methods of the invention is a heterologous immune response, such that the immune response is directed to at least one HIV isolate that is different from the HIV isolate(s) represented by the Env glycoprotein monomers in the heterotrimers administered to the host. In some embodiments, the heterologous immune response is a humeral immune response and provides antibodies, such as neutralizing antibodies and/or protective antibodies. In some embodiments, the methods of the invention elicit broadly reactive neutralizing antibodies, for example, neutralizing antibodies that are directed to at least ten different HIV isolates (such as 20, 50, or 100 different HIV isolates). In some embodiments, the heterologous immune response comprises neutralizing antibodies directed to HIV isolates from different clades.

Thus, some embodiments provide methods for eliciting an immune response in a vertebrate against HIV or an HIV-infected cell, comprising the step of administering to a vertebrate host a prophylactically or therapeutically effective amount of a composition comprising an HIV envelope heterotrimer, wherein the heterotrimer comprises at least two different Env glycoprotein monomers and wherein the composition elicits an immune response to at least one HIV isolate that is different from the HIV isolate(s) represented by the Env glycoprotein monomers in the heterotrimers.

Moreover, some embodiments provide methods for preventing or treating an HIV infection, comprising the step of administering to a vertebrate host a prophylactically or therapeutically effective amount of a composition comprising an HIV envelope heterotrimer, wherein the heterotrimer comprises at least two different Env glycoprotein monomers.
The invention also provides methods for making a vaccine composition, by suspending and packaging the heterotrimer immunogens in a suitable pharmaceutically acceptable carrier solution.
The invention further provides methods for producing neutralizing antibodies. In some embodiments, the neutralizing antibodies are produced by immunizing a host with the heterotrimers of the invention. The host may be any host capable of raising an immune response against the heterotrimers, including a mammalian host, such as a human host. In some embodiments, the neutralizing antibodies may be generated in vitro from a mammalian host that has been immunized with the heterotrimers of the invention, as has been previously described (Cole et al., 2001; Feldhaus and Siegel, 2004; Persson et al., 1991; Robinson et al., 1998; Williamson et al., 1993). In some embodiments, the methods for generating neutralizing antibodies comprise contacting antibody-producing cells with a heterotrimer of the invention.

The invention also provides neutralizing antibodies elicited by using the heterotrimers as immunogens. These neutralizing antibodies may be in serum or mucosal secretions, or an immunoglobulin fraction of serum or mucosal secretions, or may be substantially purified (monoclonal or polyclonal) using methods known in the art. Such antibodies are useful for diagnostic and/or immunotherapy purposes. In some embodiments, the neutralizing antibodies are broadly reactive, as described above. For example, the neutralizing antibodies may have a specificity that is similar to the b12 monoclonal antibody described in U.S. Patent Nos. 5,652,138 and 5,804,440, herein incorporated by reference. The affinity and specificity of the antibodies may be determined using standard methods.

The following examples illustrate representative embodiments now contemplated for practicing the invention, but should not be construed to limit the invention.

### EXAMPLE 1

This Example describes HIV-1 gp160 heterotrimers containing at least one monomer with at least one variable loop deletion.

The V1, V2, and V3 regions (individually or in combination) of the SF162 Env have been removed by mutagenesis by our group (Saunders et al., 2005; Stamatatos and Cheng-Mayer, 1998; Stamatatos et al., 2000; Stamatatos et al., 1998). DNA vectors expressing the parental and these modified proteins have been generated. These homotrimeric gp140 proteins lacking the V2 loop (dV2), the V3 loop (dV3), or both the V2 and V3 loops (dV2dV3), as well as the parental gp140 (SF162) have been used as immunogens to elicit NAbs in animals (Barnett et al., 2001; Buckner et al., 2004; Cherpelis et al., 2001; Derby et al., manuscript in preparation). Homotrimeric SF162 constructs lacking the V1 loop (dV1) have also been generated (Saunders et al., 2005).

In this Example, heterotrimers are engineered that contain SF162 derived monomers that differ in their V1, V2, V3 regions. Several combinations are possible. For example, some homotrimers may have two gp160/gp140 molecules of a given Env and a third molecule from a different Env, or they can have three different gp160/gp140 molecules. An exemplary heterotrimer is composed of two SF162 gp160/gp140 molecules and one dV3, dV2, or dV2dV3 gp160/gp140 molecule. Another exemplary heterotrimer contains one SF162 gp160/gp140 molecule, and two dV2, two dV3, or two dV2dV3 gp160/gp140 molecules. Other combinations are also possible.

SF162gp164 and either dV3gp160 or dV2gp160 have been co-expressed on the surface of viral particles. Because dV3gp160 is not capable of mediating virus-cell fusion, dV3gp160 expressing viral particles are not infectious (Saunders et al., 2005). In contrast, SF162gp160 expressing particles are. Co-expression of dV3gp160 with SF162gp160 will result in a reduction of viral infectivity if the dV3gp160 and

SF162gp160 co-exist within the same gp160 trimer (SF162/DV3 heterotrimer). The degree of reduction should be proportional to the relative concentrations of dV3gp160 and SF162gp160. Indeed, we confirmed that this is the case, which indicates that SF162 Env and dV3 Env can associate. Similarly, because the dV2gp160 is several fold less efficient in mediating infection than SF162gp160 (Saunders et al., 2005), the co-expression of dV2gp160 with SF162gp160 significantly reduces viral infectivity, an indication that dV2gp160 and SF162gp160 can form heterotrimers.

Soluble gp140 heterotrimers containing at least one more monomer with at least one variable loop deletion are being engineered using standard methodologies, for example, as described in EXAMPLE 3.

### EXAMPLE 2

This Example describes HIV-1 gp160 heterotrimers containing at least one monomer from a different clade.

DNA vectors expressing the gp164 Env proteins of the four clade A envelopes (Q168, Q259, Q461, and Q769) were provided to us by Dr. Julie Overbaugh at the FHCRC. Several modifications were introduced into these constructs. The V1 or V2 loops were eliminated using similar methodologies to those used to eliminate these loops from the SF162 Env (Saunders et al., 2005; Stamatatos and Cheng-Mayer, 1998; Stamatatos et al., 2000; Stamatatos et al., 1998). A conserved N-linked glycosylation site (NLGS) was also eliminated from the N-terrminal region of V2 (GMV2) and a conserved NLGS from the N-terminus of the V3 loop (GMV3). These modifications differentially affect the function of these Envs. Some abrogate its ability to mediate infection; some reduce it; while others do not affect it. Virions co-expressing the above-mentioned SF162 and clade A derived Envs were generated and, using similar infectivity methodologies described in EXAMPLE 1, it was shown that clade A and SF162 Env proteins can form heterotrimers.

Soluble gp140 heterotrimers containing at least one more monomer from a different clade are being engineered using standard methodologies, for example, as described in EXAMPLE 4.

### EXAMPLE 3

This Example describes the generation of soluble HIV-1 gp140 heterotrimers containing at least one more monomer with at least one variable loop deletion.

One of several methods may be used to engineer soluble gp140 heterotrimers containing at least one monomer with a variable loop deletion, for example, as described in EXAMPLE 1. In one method, a maltose binding protein (MBP) was introduced at the C-terminus of the dV3gp140 and a His tag on the C-terminus of dV2gp140. First the sequence of HCV-3C cleavage site was introduced at the C-terminus of dV3gp140 and dV2gp140. Then, a His tag sequence was introduced at the C-terminus of HCV-3C for dV2gp140, and a Maltose Binding Protein (MBP) sequence was introduced at the C-terminus of HCV-3C for dV3gp140.

Addition of the His tag at the C-terminus of HCV-3C of dV2gp140 was accomplished using Stratagene's site directed mutagenesis kit. Primers incorporating the His tag immediately downstream of the 12 nucleotide linker region were used to add six histidine residues (5' CATCACCATCACCATCAC 3', SEQ ID NO:1) to the C-terminus of the SF162dV2 gp140 construct, at the C-terminus of the newly introduced HCV-3C cleavage site. The mutagenesis reactions were carried out and transformation into chemically competent cells were done according to protocol.

The MBP tag was introduced at the C-terminal end of the HCV-3C cleavage site of SF162dV3 as follows. Using site-directed mutagenesis, EcoRI restriction sites were added to each end of the *malE* gene in the pMAL-c2x vector (New England Biolabs #E8000S). The new vector was digested with EcoRI, the male gene was isolated and ligated into the dV3gp140 expressing DNA vector at the C-terminus of the HCV-3C cleavage site.

DNA vectors expressing these proteins were generated and both proteins were shown to be expressed during transfection. Initially, the His-tagged dV2gp140 and MBP-tagged dV3gp140 constructs were transfected individually into 293T cells for 72 hours.

The His-tagged dV2gp140 containing cell supernatants were put on a cobalt affinity column (Clontech 635501). 1 ml fractions were collected following elution with a solution of 50 mM sodium phosphate and 300 mM sodium chloride, pH 5.0. Fractions containing the highest amounts of protein (as determined via Bradford assay) were pooled and subjected to Western blotting with anti-His tagged antibodies or anti-HIV Env antibodies to confirm the presence of His-tagged dV2gp140 proteins in the cell supernatant of transfected cells. The MBP-tagged dV3gp140 containing cell supernatants were incubated with an amylose resin (NEB E8021S) overnight at 4°C with agitation. The amylose resin was then packed in a column, washed, and 1 ml fractions were collected following elution with 10 mM maltose. Presence of gp140 protein was detected via Western blotting with anti-MBP and anti-HIV Env antibodies.

Subsequently, 293T cells were co-transfected with DNA vectors expressing both the His-tagged dV2gp140 and the MBP-tagged dV3gp140. The cell supernatant was first put on an anti-His column and then on an anti-MBP column, or first on an anti-MBP column and then on an anti-His column. This should result in the isolation of heterotrimeric species containing either one dV2 and two dV3 components, or two dV2 and one dV3 components.

Heterotrimers containing SF162gp140 may be purified with a modified version of the above-mentioned methodology. Because the SF162gp140 can be recognized by anti-V3 antibodies (which do not recognize the dV3gp140) and anti-V2 antibodies (which do not recognize the dV2gp140), SF162/dV3gp140 heterotrimers can be separated from all other oligomeric species by affinity column chromatography with anti-V3 MAbs and anti-MBP columns, while SF162/dV2gp140 heterotrimers can be separated from all other oligomeric species by affinity column chromatography with anti-V2 MAbs and anti-his columns.

### EXAMPLE 4

This Example describes the generation of soluble HIV-1 gp140 heterotrimers containing at least one more monomer from a different clade.

One of several methods may be used to engineer soluble gp140 heterotrimers containing at least one monomer from a different clade, for example, as described in EXAMPLE 2. In an exemplary method, the FLAG epitope is incorporated in the V4 loop of the 4 clade A Envs. A FLAG tag (5' DYKDDDDKK 3', SEQ ID NO:2) is incorporated into the V4 loop of clade A gp140s. This is accomplished by PCR- based site directed mutagenesis using Stratagene's QuikChange XL kit. Primers coding for the FLAG tag nucleotide sequence (5' GATTACAAGGATGACGATGACAAAAAG 3', SEQ ID NO:3) and specific to each isolate's V4 region are used for this process. PCR products are transformed into competent cells as per protocol, and plasmids are isolated.
DNA vectors expressing the gp140 versions of the above-mentioned clade A envelopes have been generated. The plasmids containing the FLAG-tagged clade A gp140 are transfected into 293T cells for 72 hours. The cell supernatants containing soluble gp140 are collected, and protein expression of the FLAG-tagged gp140 is conformed via Western Blotting with an anti-tag antibody. Upon confirmation of expression, the clade A plasmids are co-transfected with SF162gp140, the soluble peptides collected, and purified through an anti-FLAG column (Sigma A2220). Fractions are collected following elution via FLAG peptide competition (Sigma F3290). Fractions containing the desired proteins are affinity-purified using clade B-specific monoclonal antibodies. Detection of clade A/B gp140 heterotrimers is accomplished using Western Blotting with a clade B specific monoclonal antibody and an anti-FLAG antibody.

Similarly, clade A/B heterotrimers are generated containing the above-mentioned clade A Envs and the SF162 derived dV1, dV2, or dV3 Envs. Deletions of the V1 and V2 loops of the Q168, Q259, Q461, and Q769 isolates have been generated and these constructs are used to generate heterotrimers between them and SF162Env and its V1,

V2, and V3 modified versions mentioned above.

### Each of the references cited herein is hereby incorporated by reference.

Barnett, S. W., et al. (2001) J Virol 75, 5526-5540.
Beddows, S., et al. (1999) J Virol 73, 1740-1745.
Binley, J. M., et al. (2000) J Virol 74, 627-643.
Binley,J. M., et al. (2002) J Virol 76, 2606-2616.
Bolmstedt, A., et al. (2001) Vaccine 20, 397-405.
Buckner, C., et al. (2004) Virology 320, 167-180.
Bures, R., et aL (2000) AIDS Res Hum Retroviruses 16, 2019-2035.
Burton, D. R., et al. (1994) Science 266, 1024-1027.
Calarese, D. A., et al. (2003) Science 300, 2065-2071.
Cherpelis, S., et al. (2001) J Virol 75, 1547-1550.
Cho, M. W., et al. (2001) J Virol 75, 2224-2234.
Coffin, J. M. (1986) Cell 46, 1-4.
Cole, K. S., et al. (2001) Virology 290, 59-73.
Conley, A. J., et al. (1994) J Virol 68, 6994-7000.
Dong, M., et al. (2003) J Virol 77, 3119-3130.
Earl, P. L., et al. (2001) J Virol 75, 645-653.
Farzan, M., et al. (1998) J Virol 72, 7620-7625.
Feldhaus, M. J., and Siegel, R. W. (2004) J Immunol Methods 290, 69-80.
Gorse, G. J., et al. (1999) AIDS Res Hum Retroviruses 15, 115-132.
Grovit-Ferbas, K., et al. (2000) J Virol 74, 5802-5809.
Gzyl, J., et al. (2004) Virology 318, 493-506.
Haigwood, N. L., et al. (1992) J Virol 66, 172-182.
Hanson, C. V. (1994) AIDS Res Hum Retroviruses 10, 645-648.
Jeffs, S. A., et al. (2002) J Gen Virol 83, 2723-2732.
Kim, Y. B., et al. (2003) Virology 305, 124-137.
Kwong, P. D., et al. (2002) Nature 420, 678-682.
Kwong, P. D., et al. (1998) Nature (London) 393, 648-659.
Lifson, J. D., et al. (2004) AIDS Res Hum Retroviruses 20, 772-787.
Lorin, C., et al. (2004) J Virol 78, 146-157.
Lu, S., et al. (1998) AIDS Res Hum Retroviruses 14, 151-155.
Mascola, J. R., et al. (1996) J Infect Dis 173, 340-348.
McCaffrey, R. A., et al. (2004) J Virol 78, 3279-3295.
Moulard, M., et al. (2002) Proc Natl Acad Sci U S A 99, 6913-6918.
Muster, T., et al. (1993) J Virol 67, 6642-6647.
Nara, P. L., et al. (1988) J Virol 62, 2622-2628.
Pantophlet, R., et al. (2003) J Virol 77, 642-658.
Persson, M. A., et al, (1991) Proc Natl Acad Sci U S A 88, 2432-2436.
Purtscher, M., et al. (1996) Aids 10, 587-593.
Purtscher, M., et al. (1994) AIDS Res Hum Retroviruses 10, 1651-1658.
Quinones-Kochs, M. I., et al. (2002) J Virol 76, 4199-4211.
Remitter, J. N., et al. (1998) Nature Medicine 4, 679-684.
Robinson, J. E., et al. (1998) AIDS Res Hum Retroviruses 14, 1253-1262.
Sanders, R. W., et al. (2002a) J Virol 76, 7293-7305.
Sanders, R. W., et al (2002b) J Virol 76, 8875-8889.
Saunders, C. J., et al. (2005) J Virol 79, 9069-9080.
Scanlan, C. N., et al. (2002) J Virol 76, 7306-7321.
Schonning, K., et al. (1996) Virology 218, 134-140.
Schulke, N., et al. (2002) J Virol 76, 7760-7776.
Srivastava, I. K.; et al. (2003a) J Virol 77, 11244-11259.
Srivastava, I. K., et al. (2003b) J Virol 77,2310-2320.
Starnatatos, L., and Cheng-Mayer, C, (1998) J Virol 72, 7840-7845.
Starnatatos, L., et al. (2000) AIDS Res and Human Retroviruses 16, 981-994.
Starnatatos, L., et al, (1998) AIDS Res Hum Retroviruses 14, 1129-1139.
Stephens, D. M., et al. (1992) J Gen Virol 73, 1099-1106.
Thali, M., et al. (1993) J Virol 67, 3978-3988.
Trkola, A., et al. (1996) Nature (London) 384, 184-187,
VanCott, T. C., et al. (1999) J Virol 73, 4640-4650.
Williamson, R. A., et al. (1993) Proc Natl Acad Sci U S A 90, 4141-4145.
Wyatt, R., et al. (1998) Nature (London) 393, 705-711.
Wyatt, R., et al, (1995) J Virol 69, 5723-5733.
Wyatt, R., et al. (1993) J Virol 67, 4557-4565.
Xiang, S. H., et al. (2002) AIDS Res Hum Retroviruses 18, 1207-1217.
Yang, X., et al. (2000) J Virol 74, 4746-4754.
Yang, X., et al, (2001) J Virol 75, 1165-1171.
Yang, X., et al. (2004a). J Virol 78, 12975-12986.
Yang, Z. Y., et al. (2004b) J Virol 78, 4029-4036.
Zwick, M. B., et al. (2001) J Virol 75, 10892-10905.

While the preferred embodiment of the invention has been illustrated and described, it will be appreciated that various changes can be made therein without departing from the spirit and scope of the invention.

The following paragraphs form part of the description:
1. An HIV envelope heterotrimer, comprising three different Env glycoprotein monomers, wherein at least two Env glycoprotein monomers are from different HIV isolates.
2. The heterotrimer of paragraph 1, wherein the different HIV isolates comprise one or more HIV-1 isolates.
3. The heterotrimer of paragraph 2, wherein the one or more HIV-1 isolates belong to different clades.
4. The heterotrimer of paragraph 1, wherein at least one of the Env glycoprotein monomers comprises a deletion of a variable loop region.
5. The heterotrimer of paragraph 1, wherein at least one of the Env glycoprotein monomers comprises an alteration of a glycosylation site.
6. A vaccine comprising an effective amount of a composition comprising an HIV envelope heterotrimer and a pharmaceutically acceptable carrier, wherein the HIV envelope heterotrimer comprises at least two different Env glycoprotein monomers.
7. A method for inducing an immune response in a vertebrate host against HIV or an HIV-infected cell, comprising administering to a vertebrate host a prophylactically or therapeutically effective amount of a composition comprising an HIV envelope heterotrimer, wherein the heterotrimer comprises at least two different Env glycoprotein monomers.
8. The method of paragraph 7, wherein the induced immune response comprises broadly reactive neutralizing antibodies.

## Claims

1. An HIV envelope heterotrimer, comprising at least two different Env glycoprotein monomers, wherein at least two Env glycoprotein monomers are from different HIV isolates.

2. The heterotrimer of claim 1, wherein the different HIV isolates comprise one or more HIV-1 isolates.

3. The heterotrimer of claim 2, wherein the one or more HIV-1 isolates belong to different clades,

4. The heterotrimer of claim 1, wherein at least one of the Env glycoprotein monomers comprises a deletion of a variable loop region.

5. The heterotrimer of claim 1, wherein at least one of the Env glycoprotein monomers comprises an alteration of a glycosylation site.

6. An HIV envelope heterotrimer as defined in any one of claims 1 to 5 for inducing an immune response in a vertebrate host against HIV or an HIV-infected cell.
